# EUROPEAN PATENT SPECIFICATION

(11) **EP 0 996 391 B1**
(45) Date of publication and mention of the grant of the patent: **11.02.2004**
(21) Application number: 98928742.0
(22) Date of filing: 02.06.1998
(51) Int. Cl.: A61F 2/20, A61C 8/00, H04R 25/02

(54) **A DEVICE FOR ANCHORING AND ENERGY TRANSFER AT IMPLANTS**
BEFESTIGUNGS- UND ENERGIEÜBERTRAGUNGS- VORRICHTUNG FÜR IMPLANTATE
SYSTEME D'ANCRAGE ET DE TRANSFERT D'ENERGIE DESTINE A DES IMPLANTS

(30) Priority: 06.06.1997 SE 9702164
(43) Date of publication of application: 03.05.2000
(73) Proprietor: P & B RESEARCH AB, 412 96 Göteborg (SE)
(72) Inventor: HaKANSSON, Bo, S-416 80 Göteborg (SE); CARLSSON, Peder, S-442 93 Kungälv (SE)
(74) Representative: Westman, Per Börje Ingemar
(86) International application number: PCT/SE1998/001049
(87) International publication number: WO 1998/055049

(56) References cited:
- EP-A- 0 715 839
- WO-A-97/13477
- US-A- 4 328 813
- US-A- 4 629 451

## Description

### TECHNICAL FIELD

The present invention refers to a device at implants for anchoring in bone tissue and supporting of a prosthesis or transfer of electrical and/or mechanical energy from a transmitter or the like to the implant via a coupling device, which incorporates a first and a second coupling part, and wherein the implant incorporates a flange fixture.

### BACKGROUND AND TECHNICAL PROBLEM OF THE INVENTION

An example of such implants is for instance skull bone anchored implants for transfer of signals from a hearing aid connected to the implant, wherein the flange fixture is made integral with the first coupling part, forming a continuous unit, whereby the second coupling part with one of its ends is connectable to said prosthesis, transmitter or the like and with its other end is connectable to the first coupling part. More specifically the invention can constitute a development of the device described in EP-A-715839 (SE-C2 503790).

For such applications today is used a two-piece implant, the parts of which are affixed to each other by means of a screw joint. One part of the implant consists of a threaded element, which is anchored in the bone tissue and the second part is a socket, which penetrates the soft tissue. The reason for the implant used today to be in two pieces is i.a. that the surgical technique used today requires an operation made in two steps. In the first step, the threaded element or the bone screw is installed. This is thereupon allowed to heal up during a time period of several months without being subjected to any external stresses during that time. Not until after this healing period the second part of the operation is carried through, whereby the external skin penetrating socket is installed.

Furthermore the mutual rotational affixing between the bone anchored inner element and the skin penetrating outer part can be adjusted, which is necessary at certain types of couplings, such as bayonet couplings. In case of possible, more serious skin irritations, the outer socket can be removed without influencing the bone anchored element.

The connecting screw is also acting as an overload protection, which can be designed to burst at heavy, external mechanical stresses, in order not to risk that the healed up, bone anchored element shall be broken away.

Due to the two-piece design it is possible to up-grade the coupling device used without need of removal of the bone screw, and if the external skin penetrating socket is damaged it is also possible to exchange it without influence on the bone screw.

If the patient finally is not satisfied with the member connected to the implant (e.g. a hearing aid), the intact skin can be restored without need for removal of the bone anchored element.

A problem connected to the hitherto used two-piece implants is that the manufacturing costs will become rather high, on one hand due to the number of parts, i.e. bone screw, outer socket, connecting screw, and often also sealing and covering members, and on the other hand due to the requirement for very high tolerances for the internal fitting of the parts concerned, in particular for signal transferring applications.

### PURPOSE OF THE INVENTION AND SOLUTION OF THE PROBLEM

The purpose of the present invention is to create an implant of the type mentioned in the introductory part, which:
has a lower height and therefore is less subjected to external influence, consists of fewer elements,
is simpler and less expensive to manufacture with lower demand for high tolerances,
has a lower frequency of skin irritation,
simplifies the surgical procedure and cuts the healing time substantially, shall have ability to take up a certain external force without the implant coming loose.

Furthermore the advantages inherent in the known implant shall be maintained or even be improved, which means:
that at more serious cases of skin irritation or at damages on the skin penetrating outer part, it shall be possible to remove this without influencing the inner, bone anchored part,
that interconnection function by means of a snap action connection and disconnection by means of a turning motion is maintained.

These tasks have been solved by the features defined in the claims.

### DESCRIPTION OF THE DRAWINGS

Hereinafter the invention will be described more in detail in some embodiments with reference to the accompanying drawings.
Fig. 1 shows a longitudinal section through a first embodiment of an implant according to the invention with added apparatus coupling part.
Fig. 2 shows a section through the implant rotated 90° in comparison to Fig. 1, after disconnection of its coupling part, reinstallment thereof and screw tightening thereof.
Fig. 3 shows a section through an implant according to a second embodiment.
Fig. 4 shows a section through all the elements forming part of the second embodiment according to Fig. 3, after disconnection of the implant screw.
Fig. 5 shows a section through the assembled implant according to Fig. 4, with mounted apparatus coupling part.
Figs. 6 and 7 show sections through a third and a fourth embodiment of the invention.
Fig. 8 shows a section through a separate first coupling part.

### DESCRIPTION OF EMBODIMENTS

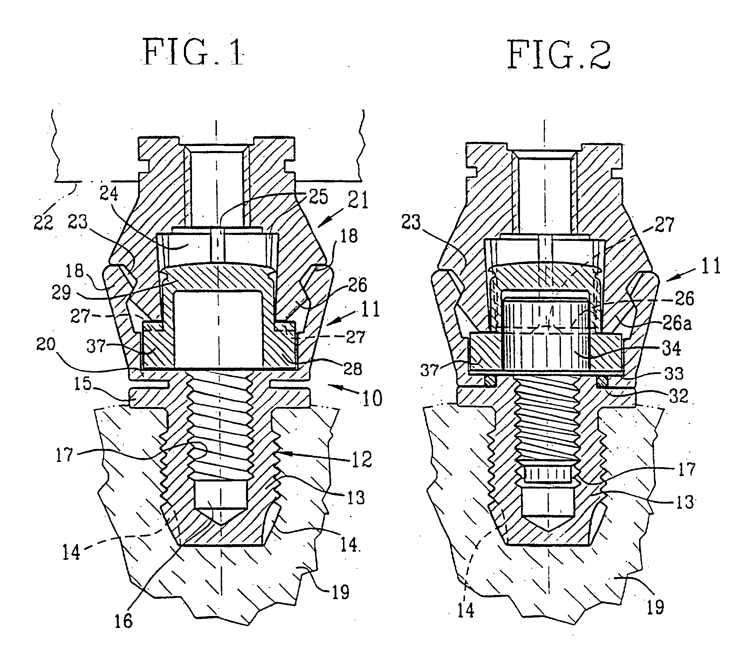

Figs. 1 and 2, which show a first embodiment of the device according to the invention illustrate this in two different stage, which hereinafter will be described more in detail.

In contrast to the coupling device according to SE-C2-503790 the coupling part 11 of the implant 10 is made integral with the flange fixture 12 of the implant, which fixture consists of a screw 13, which at its lower part is equipped with a chamfer 14, which possibly can form cutting edges, whereas the upper part of the screw continues in a flange 15. Furthermore the flange fixture is provided with an axial blind bore 16 with internal threads 17. The implant continues from the flange 15 and continues in a coupling part 11, hereinafter referred to as the coupling part of the implant, which is formed as a socket and which at its upper, free end edge at the inner side is equipped with an annular bulge 18. The implant is manufactured from a material which is satisfactorily accepted by the human body, e.g: titanium or stainless steel, which means that the socket shaped coupling part 11 is rigid.

The screw 13 of the implant 10 is intended to be screwed into a bore in bone tissue 19, which for instance can be the skull bone, whereby the screw is tightened so deeply that the flange 15 will rest against the bone tissue.

The transition between the flange fixture 12 of the implant an its coupling part 11 is designed as a material weakening 20, which forms a deformation zone.

The second coupling part 21 of the device, hereinafter also referred to as the apparatus part, is connectable with one of its ends to a hearing aid 22, a prosthesis or the like and the other end of which is designed as a male part, which is connectable to the female-shaped coupling part 11 of the implant. For this purpose the apparatus part is designed with a circumferential groove 23, intended to cooperate by snap action with the annular bulge 18 at the coupling part 11 of the implant. The apparatus part 21 is provided with an internal recess 24, whereby also this part is socket-shaped. The resilience of the male part has been achieved by means of a number of axial slots 25, whereby is formed resilient tongues 26. At least some of the tongues 26a are longer than the rest of the tongues 26, which longer tongues at turning of the apparatus part 21 cooperate with at least one ridge-like, raised portion 27, which in the embodiment shown is arranged on the circumferential flange 28 of a releaving member 29, which is partly insertable into the recess 24.

As the two coupling parts 11 and 21 are mutually rotatable, at such a rotation the longer tongues 26a will slide up on the ridge 27, whereby the coupling parts will be disconnected from each other.

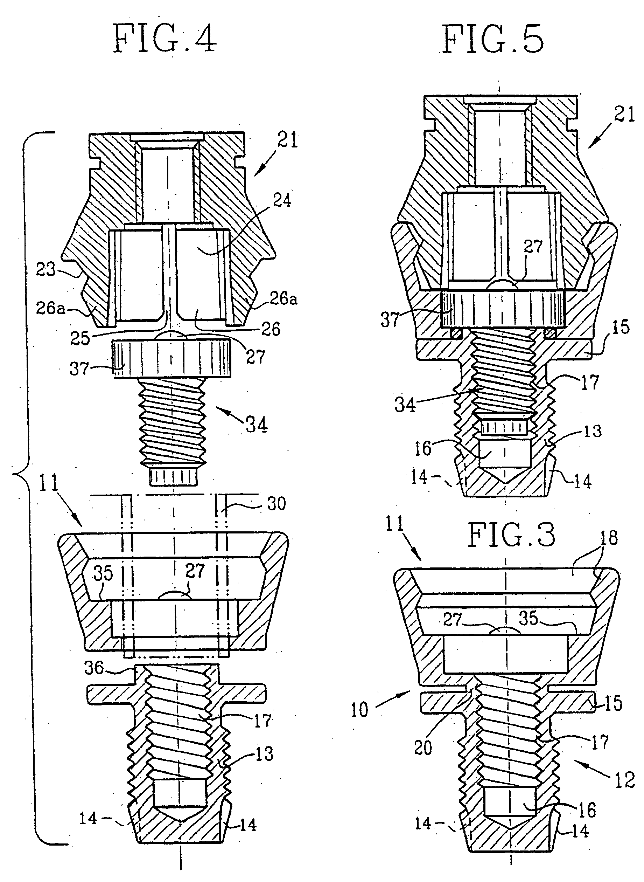

If, for any reason, the coupling part 11 of the implant must be removed, e.g. due to skin irritation or due to the fact that it has become damaged or disformed, this can be effected by means of a socket cutter 30, (shown in Fig. 4), whereby a part of the deformation zone 20 can be cut away thus that the coupling part 11 and the apparatus part 21 of the implant are separated. If the couping part 11 of the implant is not damaged it can be re-used, whereby a sealing ring 32 is positioned in the annular space 31 created by the socket cutter 30, whereupon, by means of a washer 33 and a connecting screw 34, which is screwed into the internal threads 17 of the implant screw 13, is achieved a connection between the parts 11 and 21.

Due to the fact that the coupling part 11 and the flange fixture 12 of the implant are made integral, the operation can be carried out in one stage and be used to full extent after a healing period of a few weeks . Disconnection of the two coupling parts 11 and 21 can be effected in the same simple manner as earlier, by rotating the apparatus part relative to the stationary implant.

Figures 3, 4 and 5 show a somewhat modified embodiment, wherein the release member 29 is redundant and the ridge-like raised portions 27 are arranged at a shoulder 35 inside the socket-shaped coupling part 11. The connecting screw 34 can be designed with such a wide head, that the released first coupling part 11, without intermediary connection of a washer can be screwed together, thereby creating a good physical contact between the parts. By this design the number of components used is reduced with one further part.

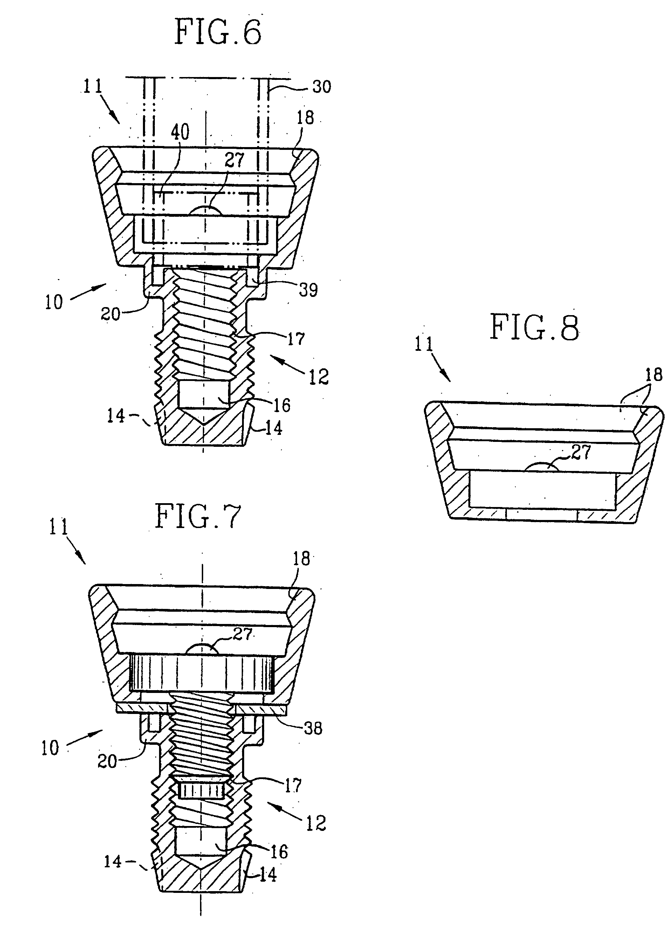

Figs. 6 and 7 show an embodiment having a somewhat modified deformation zone, consisting of a transistion from the first coupling part 11 to the flange fixture 12, having a Z-shaped cross section. In the same manner as in the previous embodiment, this zone shall have an ability to take up external influence upon the first coupling part in such a manner that only a minor portion of these stresses are transferred elastically to the flange fixture. A portion of the deformation zone 20, e.g. the web of the Z-profile, can be milled away at a necessary dismounting of the main parts 11, 12 of the implant, thus that the parts are separated, with the flange fixture still being affixed in the bone tissue. In order to be able to re-connect these parts it is required a washer 38, which bridges the milled away portion. Thus the lower edge of the first coupling part 11 thereby will be screwed against the washer 38, which can consist of an elastic material, thus that a deformation zone is created after the separation and the subsequent re-connection of the parts. The annular groove 39 between the deformation zone 20 and the flange fixture 12 can be used as a groove for a guiding pin 40 provided at the end of the cutter 30.

Of course it is also possible to substitute the deformed and separated first coupling part 11 for a separate such part, such as shown in Fig. 8, and which does not need to be completed by some loose details, such as the washer 38, as this is built into the coupling part.

The invention is not limited to the embodiments described, but a number of variants are possible within the scope of the claims. Thus the first coupling part of the implant, which in the embodiments shown, is designed as a female part intended to receive the male-shaped second coupling part, can instead be designed as a male part whereas the second coupling part is designed as a female part.

### LIST OVER REFERENCE NUMERALS

- 10 =: Implant
- 11 =: First coupling part
- 12 =: Flange fixture
- 13 =: Screw
- 14 =: Chamfer
- 15 =: Flange
- 16 =: Blind bore
- 17 =: Internal threads
- 18 =: Annular bulge
- 19 =: Bone tissue
- 20 =: Material weakening / Deformation zone
- 21 =: 2nd coupling part / Apparatus part
- 22 =: Hearing aid
- 23 =: Groove
- 24 =: Recess
- 25 =: Slots
- 26 =: Short tongue
- 26a =: Long tongue
- 27 =: Raised portion/ ridge
- 28 =: Flange
- 29 =: Release member
- 30 =: Socket cutter
- 31 =: Annular space
- 32 =: Sealing ring
- 33 =: Washer
- 34 =: Connecting crew
- 35 =: Shoulder
- 36 =: Neck
- 37 =: Flange
- 38 =: Washer
- 39 =: Annular groove
- 40 =: Guiding pin

## Claims

1. A device at implants (10) for anchoring in bone tissue (19) and supporting a prosthesis or transfer of electrical and/or mechanical energy from a transmitter (22) to the implant via a coupling device, which incorporates a first and a second coupling part (11, 21), and wherein the implant (10) incorporates a flange fixture (12),
which flange fixture (12), is made integral with the first coupling part (11), forming a continuous unit, whereby the second coupling part (21) with one of its ends is connectable to said prosthesis, transmitter (22) or the like and with its other end is connectable with the first coupling part (11),
**characterized therein,**
that a deformation zone (20) is provided at the transition between the flange fixture (12) and the first coupling part (11).

2. A device according to claim 1,
**characterized therein**,
that the deformation zone (20) is arranged at some distance from and substantially in parallel with the flange (15) of the flange fixture, and
that the deformation zone at the same time is a dismounting zone, within which the flange fixture is separable from the first coupling part, by means of a tool (30) insertable therein.

3. A device according to claim 2,
**characterized therein**,
that the coupling part is separable from the flange fixture, which is affixed to the bone tissue, preferably via a cylindric cutter (30).

4. A device according to claim 2,
**characterized therein,**
that the flange fixture (13) is provided with a central, axial blind bore (16) having internal threads (17), and that the separated or a new first coupling part (11) is attachable to the flange fixture (13) and connectable to this by means of a connecting screw (34), which can be screwed into the said blind bore.

5. A device according to claim 1,
**characterized therein**,
that in the interior of the socket-shaped first coupling part (11) is attachable to a release member (29) equipped with a circumferential flange (37), on which is provided at least one ridge-like raised portion (27), for cooperation with the end of a tongue (26a) at the second coupling part (21).

6. A device according to claim 1,
**characterized therein,**
that the interior of the socket-shaped first coupling part (11) is equipped with a shoulder (35) upon which is formed at least one ridge-like raised portion (27) for cooperation with the end of at least one tongue (26a) at the second coupling part (21).

## Patentansprüche

1. Vorrichtung an Implantaten (10) zum Verankern in Knochengewebe (19) und zum Tragen einer Prothese oder zum Übertragen von elektrischer und/oder mechanischer Energie von einem Sender (22) zu dem Implantat über eine Kupplungsvorrichtung, die ein erstes und ein zweites Kupplungsteil (21) enthält, und wobei das Implantat (10) eine Flanschbefestigung (12) enthält,
wobei die Flanschbefestigung (12) integral mit dem ersten Kupplungsteil (11) ausgebildet ist, eine durchgehende Einheit bildet, so dass das zweite Kupplungsteil (21) mit einem seiner Enden mit der Prothese, dem Sender (22) oder dergleichen verbunden werden kann und mit seinem anderen Ende mit dem ersten Kupplungsteil (11) verbunden werden kann,
**dadurch gekennzeichnet, dass:**
eine Verformungszone (20) an dem Übergang zwischen der Flanschbefestigung (12) und dem ersten Kupplungsteil (11) vorhanden ist.

2. Vorrichtung nach Anspruch 1, **dadurch gekennzeichnet, dass:**
die Verformungszone (22) in einem bestimmten Abstand zu dem Flansch (15) der Flanschbefestigung und im Wesentlichen parallel dazu angeordnet ist, und
die Verformungszone gleichzeitig eine Demontagezone ist, in der die Flanschbefestigung mittels eines Werkzeugs (30) von dem ersten Kupplungsteil getrennt werden kann, das in sie eingeführt werden kann.

3. Vorrichtung nach Anspruch 2, **dadurch gekennzeichnet, dass**:
das Kupplungsteil von der Flanschbefestigung, die an dem Knochengewebe befestigt ist, vorzugsweise über einen zylindrischen Schneider (30) getrennt werden kann.

4. Vorrichtung nach Anspruch 2, **dadurch gekennzeichnet, dass**:
die Flanschbefestigung (13) mit einer mittigen, axialen Sackbohrung (16) mit Innengewinde (17) versehen ist und das getrennte oder ein neues erstes Kupplungsteil (11) an der Flanschbefestigung (13) angebracht werden kann und mit dieser mittels einer Verbindungsschraube (34) verbunden werden kann, die in die Sackbohrung eingeschraubt werden kann.

5. Vorrichtung nach Anspruch 1, **dadurch gekennzeichnet, dass**:
der Innenraum des buchsenförmigen ersten Kupplungsteils (11) an einem Löseelement (29) angebracht werden kann, das mit einem Umfangsflansch (37) versehen ist, an dem wenigstens ein wulstartiger erhabener Abschnitt (27) zum Zusammenwirken mit dem Ende einer Zunge (26a) an dem zweiten Kupplungsteil (21) vorhanden ist.

6. Vorrichtung nach Anspruch 1, **dadurch gekennzeichnet, dass**:
der Innenraum des buchsenförmigen ersten Kupplungsteils (11) mit einem Absatz (35) versehen ist, an dem wenigstens ein wulstartiger erhabener Abschnitt (27) zum Zusammenwirken mit dem Ende wenigstens einer Zunge (26a) an dem zweiten Kupplungsteil (21) ausgebildet ist.

## Revendications

1. Dispositif pour implants (10) destiné à ancrer dans les tissus osseux (19) et à supporter une prothèse ou au transfert d'énergie électrique et/ou mécanique à partir d'un émetteur (22) à destination de l'implant par l'intermédiaire d'un dispositif de liaison, qui incorpore une première et une seconde partie de liaison (11,21), et dans lequel l'implant (10) incorpore un élément de bride (12),
lequel élément de bride (12) est rendu solidaire de la première partie de liaison (11), formant une unité continue, moyennant quoi la seconde partie de liaison (21) est raccordable par l'une de ses extrémités à ladite prothèse, émetteur ou analogue (22) et par son autre extrémité elle est raccordable à la première partie de liaison (11),
**caractérisé en ce que**,
une zone de déformation (20) est prévue au niveau de la transition entre l'élément de bride (12) et la première partie de liaison (11).

2. Dispositif selon la revendication 1,
**caractérisé en ce que**,
la zone de déformation (20) est disposée à une certaine distance de et parallèlement à la bride (15) de l'élément de bride, et
**en ce que** la zone de déformation en même temps est une zone de démontage, à l'intérieur de laquelle l'élément de bride est séparable de la première partie de liaison, au moyen d'un outil (30) pouvant y être inséré.

3. Dispositif selon la revendication 2,
**caractérisé en ce que**,
la partie de liaison est séparable de l'élément de bride qui est fixé sur le tissu osseux, de préférence par l'intermédiaire d'un élément de coupe cylindrique (30).

4. Dispositif selon la revendication 2,
**caractérisé en ce que**,
l'élément de bride (13) est muni d'un alésage borgne central, axial (16) ayant un filetage interne (17) et **en ce que** la partie de liaison séparée ou une nouvelle partie de liaison (11) peut être fixée sur l'élément de bride (13) et être raccordée à celui-ci au moyen d'une vis de raccordement (34) qui peut être vissée dans ledit alésage borgne.

5. Dispositif selon la revendication 1,
**caractérisé en ce que**,
à l'intérieur de la première partie de liaison en forme de douille (11) peut être fixé un élément de libération (29) muni d'une bride circonférentielle (37) sur laquelle est prévue au moins une portion surélevée en forme de nervure (27) destinée à coopérer avec l'extrémité d'une languette (26a) au niveau de la seconde partie de liaison (21).

6. Dispositif selon la revendication 1,
**caractérisé en ce que**,
l'intérieur de la première partie de liaison en forme de douille (11) est muni d'un épaulement (35) sur lequel est formée au moins une portion surélevée en forme de nervure (27) destinée à coopérer avec l'extrémité d'au moins une languette (26a) au niveau de la seconde partie de liaison (21).
